# EUROPEAN PATENT SPECIFICATION

(11) **EP 1 819 653 B1**
(45) Date of publication and mention of the grant of the patent: **16.04.2008**
(21) Application number: 05798486.6
(22) Date of filing: 01.11.2005
(51) Int. Cl.: C07C 29/151

(54) **PROCESS FOR THE PRODUCTION OF METHANOL**
VERFAHREN ZUR HERSTELLUNG VON METHANOL
PROCEDE DE PRODUCTION DE METHANOL

(30) Priority: 09.12.2004 GB 0427022
(43) Date of publication of application: 22.08.2007
(73) Proprietor: INEOS EUROPE LIMITED, Lyndhurst, Hampshire, SO43 7FG (GB)
(72) Inventor: BELL, Peter, Simpson, Dunblane FK15 9BB (GB)
(74) Representative: King, Alex
(86) International application number: PCT/GB2005/004212
(87) International publication number: WO 2006/061554

(56) References cited:
- US-A- 4 413 153
- US-A- 5 063 250
- US-A1- 2002 143 220
- US-A1- 2003 083 391

## Description

The present invention relates to a process for the production of methanol, and, in particular, to a process for the production of methanol from carbon monoxide and hydrogen in a fuel gas stream which has been separated from the product stream obtained from a process for the autothermal cracking of paraffinic hydrocarbons with oxygen in the presence of a catalyst capable of supporting combustion beyond the fuel rich limit of flammability.

The production of methanol from carbon monoxide and hydrogen-containing feed streams (synthesis gas) is well-known in the art, and is described, for example, in the Article titled "Methanol" in Ullmann's Encyclopedia of Industrial Chemistry, 2002.

The overall reaction may be represented stoichiometrically as:

CO + 2H₂ ↔ CH₃OH (Eqn. 1)

However, in practise, other reactions are believed also to be important, including:

CO₂ + 3H₂ ↔ CH₃OH + H₂O (Eqn. 2)

CO + H₂O ↔ CO₂ + H₂ (Eqn. 3)

In the general industrial process synthesis gas containing predominantly carbon monoxide and hydrogen, but also quantities of other components such as methane and carbon dioxide is passed over a methanol synthesis catalyst. The one-pass conversion of the CO & CO2 within the synthesis gas is typically approximately 50% because thermodynamic equilibrium is reached, and therefore, after methanol and water are condensed out, the remaining unreacted synthesis gas is recycled to the reactor.

It is necessary to take a purge from this recycle stream to prevent build-up of inert components therein, such as methane, and this also leads to a loss of hydrogen and carbon monoxide components, unless such components are subsequently recovered (with the associated cost of this). It is therefore generally desired to reduce the volume of the purge as much as possible by reducing the amount of inert gases such as methane in the synthesis gas feed to the process.

The crude methanol produced by this process typically comprises significant quantities of water (up to 20 to 30% by weight) which needs to be removed from the methanol. This can be done by distillation, but because of the volume of water to separate the distillation requires a large column and is very energy intensive.

Numerous processes are known for the cracking of a hydrocarbon feedstock to produce olefins. One such process is autothermal cracking, in which a paraffinic hydrocarbon feed is mixed with oxygen and passed over an autothermal cracking catalyst. Combustion is initiated on the catalyst surface and the heat required to raise the reactants to process temperature and to carry out the endothermic cracking process is generated in situ. The product stream from the autothermal cracking process typically produces a gaseous stream comprising one or more olefins, oxygenates, carbon dioxide, methane, hydrogen and carbon monoxide. Such a process is described, for example, in EP-332289B; EP-529793B; EP-0709446A, WO 00/14035 and WO 00/15587.

The olefins produced may themselves be used as feedstocks for olefin derivative processes, such as polymerization processes to produce polyethylene, polypropylene and other polymers. Components such as methane, hydrogen and carbon monoxide are typically recovered as a "fuel gas" which is burnt to provide energy for other parts of the process.

However, it would be desirable to derive better value from these components.

In particular, WO 00/15587 describes a process in which the synthesis gas components from an autothermal cracking process are subsequently separated from the olefinic products and either (i) converted to methanol, (ii) reacted in a water-gas shift reaction or (iii) converted to hydrocarbons. However, although this discloses generally that such synthesis gas may be reacted to produce methanol, there is no teaching in WO 00/15587 that this may be done without removing as much as possible of the methane in said stream, as would be expected for conventional methanol production.

In contrast to the conventional processes for methanol production, we have now found a process wherein methanol may advantageously be produced from the fuel gas stream produced from an autothermal cracking reactor without it being necessary to pretreat this stream to reduce the methane therein.

Thus, in a first aspect, the present invention provides a process for the production of methanol, said process comprising:
(a) providing a fuel gas stream comprising 20-50mol% hydrogen, 20-50mol% carbon monoxide and 20-50mol% methane, which fuel gas stream has been separated from the product stream obtained from a process for the autothermal cracking of paraffinic hydrocarbons with oxygen in the in the presence of a catalyst capable of supporting combustion beyond the fuel rich limit of flammability,
(b) contacting the fuel gas stream comprising 20-50mol% hydrogen, 20-50mol% carbon monoxide and 20-50mol% methane obtained from step (a) with a catalyst for the conversion of synthesis gas to methanol at a pressure in the range 50 to 125 barg, to produce a product stream comprising methanol, carbon monoxide, hydrogen and methane,
(c) treating the product stream comprising methanol, carbon monoxide, hydrogen, and methane from step (b) to separate a product stream comprising methanol and a process stream comprising carbon monoxide, hydrogen and methane,
(d) contacting the process stream comprising carbon monoxide, hydrogen and methane from step (c) with a water-gas shift catalyst to convert at least some of the carbon monoxide to produce additional hydrogen, and
(e) separating at least a portion of the hydrogen and recycling to step (b).

The fuel gas stream in step (a) comprises 20-50mol% hydrogen, 20-50mol% carbon monoxide and 20-50mol% methane. The stream also comprises essentially no carbon dioxide, since carbon dioxide is removed from the ATC product stream before the separation steps in which the fuel gas stream is recovered.

More typically, the fuel gas stream in step (a) comprises 30-40mol% hydrogen, 25-35mol% carbon monoxide and 30-40mol% methane.

Generally, the fuel gas stream in step (a) comprises less than the stoichiometric molar ratio of hydrogen to carbon monoxide for methanol production of 2:1 (Equation 1), typically having a molar ratio of hydrogen to carbon monoxide in the range 1:1 to 1.5:1.

Most preferably, the fuel gas stream comprising 20-50mol% hydrogen, 20-50mol% carbon monoxide and 20-50mol% methane, which fuel gas stream has been separated from the product stream obtained from a process for the autothermal cracking of paraffinic hydrocarbons with oxygen in the in the presence of a catalyst capable of supporting combustion beyond the fuel rich limit of flammability, is used directly as separated from the autothermal cracking product stream without any additional treatment steps.

The fuel gas stream according to the process of the present invention has a significantly different composition to the synthesis gas streams conventionally used for methanol production, which may be derived from steam reforming of hydrocarbons, such as methane, for example.

In particular, the fuel gas stream according to the process of the present invention comprises a significantly larger quantity of methane than would conventionally be in a synthesis gas fed to a methanol synthesis catalyst. Despite this, the process of the present invention has the advantage that there is no requirement for methane removal from the fuel gas prior to feeding to the methanol synthesis catalyst.

In addition, the fuel gas stream comprises more carbon monoxide and less hydrogen, and therefore also a lower hydrogen to carbon monoxide ratio than would conventionally be in a synthesis gas stream used for methanol production. Steam reforming of hydrocarbons, for example, generally would give a mixture with significantly more hydrogen than the stoichiometric hydrogen to carbon monoxide ratio of 2:1.

Finally, the fuel gas stream according to the process of the present invention comprises essentially no carbon dioxide, whereas synthesis gas from other sources generally comprises significant quantities of carbon dioxide.

In the process of the present invention, the fuel gas stream is reacted in a "once-through" methanol synthesis process, and carbon monoxide in the product stream from the methanol synthesis process is subsequently used to produce additional hydrogen which can be recycled. The recycled hydrogen acts to increase the hydrogen to carbon monoxide ratio relative to that in the original fuel gas. Thus, the hydrogen to carbon monoxide ratio of the reactants with which the catalyst is actually contacted (i.e. the mixture of fuel gas and recycle stream) can be controlled, using the hydrogen recycle. According to the process of the present invention therefore, the molar ratio of hydrogen to carbon monoxide is increased from that in the original fuel gas, preferably to approximately the stoichiometric 2:1 molar ratio, by addition of hydrogen itself derived from the carbon monoxide present in the product stream from the methanol synthesis. Effectively, this allows higher (total) carbon monoxide conversions to be achieved (conversion takes place in the once-through methanol step and extra conversion in hydrogen production) without the need for carbon monoxide recycle. The once-through process of the present invention provides a relatively simple and cost-efficient means of recovering some of the value from the fuel gas stream.

Gas composition in a conventional methanol loop usually comprises significantly more than a 2:1 ratio of hydrogen to carbon monoxide. Whilst favouring increased carbon monoxide (carbon dioxide) conversion and increased methanol production, increased hydrogen in the feed also favours increased water production, due to the presence of carbon dioxide (Eqn. 3), which is why the methanol produced will typically contain up to 30% by weight of water.

In the process of the present invention, the concentration of hydrogen in the fuel gas stream, and hence in the methanol synthesis reaction (i.e. even after combination with recycle hydrogen components), is lower than when conventional synthesis gas/recycle is used. There are also only small amounts of carbon dioxide present in the contacting step (b) (as described further below). Thus, the product stream comprising methanol produced will typically contain significantly less water than in conventional methanol synthesis, such as less than 5wt% water. Therefore, purification of the crude methanol will be significantly less energy intensive. Sale or further use of the methanol without purification may also be possible (depending on the market/use).

Although the fuel gas stream comprising 20-50mol% hydrogen, 20-50mol% carbon monoxide and 20-50mol% methane, which fuel gas stream has been separated from the product stream obtained from a process for the autothermal cracking of hydrocarbons comprises essentially no carbon dioxide, it is generally desirable to include a small amount of carbon dioxide in the methanol synthesis step (b). This is because, although the stoichiometric reaction for methanol synthesis is that given by Eqn. 1 above, the reaction is believed to operate at least in part through the reactions in Eqns. 2 and 3 above, in which carbon dioxide reacts to produce methanol and water, and the water then reacts with carbon monoxide to regenerate the carbon dioxide and some of the hydrogen. Carbon dioxide may be added separately to the methanol synthesis step (b) or may be produced in the methanol synthesis step (b) by feeding small amounts of water which will react to produce carbon dioxide, but most preferably, carbon dioxide is recycled with the hydrogen in step (e), since carbon dioxide is also produced by the water-gas shift reaction of carbon monoxide in step (d) (carbon monoxide and water react to produce hydrogen and carbon dioxide, as described by Eqn. 3).

In step (b) of the process of the present invention, the fuel gas stream comprising 20-50mol% hydrogen, 20-50mol% carbon monoxide and 20-50mol% methane obtained from step (a) is contacted with a catalyst for the conversion of synthesis gas to methanol at a pressure in the range 50 to 125 barg, to produce a product stream comprising methanol, carbon monoxide, hydrogen and methane. The product stream will also typically comprise smaller amounts of carbon dioxide and water.

The catalyst may be contained in, and hence the contacting takes place in, any suitable methanol synthesis reactor.

The recycle stream comprising hydrogen from step (e) may passed to the methanol synthesis reactor as a separate stream or may be combined with the fuel gas stream prior to passing the combined stream to the methanol synthesis reactor.

The reaction of step (b) is preferably performed at a pressure in the range 50 to 100 barg. Lower pressure methanol synthesis is generally economically preferred over higher pressure processes, and is described, for example, in the Article titled "Methanol" in Ullmann's Encyclopedia of Industrial Chemistry, 2002.

The reaction temperature is typically maintained in the range 200-300°C.

Any suitable catalyst may be used. Preferred catalysts are typically based on copper, and especially on copper and zinc oxides. Suitable catalysts are described, for example, in US 4,596,782, US 4,535,071, US 3,709,919, GB 1,159,035 and Applied Catalysis 25 (1986) 109-194.

In step (c) the product stream comprising methanol, carbon monoxide, hydrogen and methane from step (b) is treated to separate a product stream comprising methanol and a process stream comprising carbon monoxide, hydrogen and methane. This may be done by any suitable method, typically by condensation of the methanol. The product stream comprising methanol typically comprises the majority of any water present in the product stream comprising methanol, carbon monoxide, hydrogen and methane from step (b). As described above, the product stream comprising methanol will typically contain less than 5wt% water. The product stream comprising methanol may be used (or sold) without further purification, or may be purified to remove any water therein.

The remaining process stream comprises carbon monoxide, hydrogen and methane. The process stream may also comprise any carbon dioxide present in the product stream and a small amount of water.

In step (d) of the process of the present invention, this process stream is contacted with a water-gas shift catalyst to convert at least some of the carbon monoxide to hydrogen. Water is added to the process stream to push the water-gas shift equilibrium towards hydrogen and carbon dioxide (Eqn. 3).

The water-gas shift reaction is also well-known in the art. Lower temperatures push the equilibrium towards hydrogen and carbon dioxide whereas higher temperatures favour carbon monoxide and water. Thus, the reaction is preferably operated at lower temperatures, typically at a temperature in the range 200-400°C.

Generally, any suitable pressure may be used since the equilibrium is not strongly dependent on pressure. However, the preferred pressure will be in the range 50 to,125 barg, these pressures being preferable for integration with the upstream methanol synthesis step and the downstream hydrogen separation step. Any suitable catalyst may be used for the water-gas shift reaction, such as an iron oxide catalyst.

The water-gas shift reaction produces a product stream comprising carbon monoxide, hydrogen, carbon dioxide, water and methane.

In step (e) at least a portion of the hydrogen in this product stream is separated and recycled (as a recycle stream) to step (b). The separation may be by any suitable means, membrane separation or pressure swing absorption being particular examples.

The recycle stream comprises hydrogen and will typically (and preferably, as described above) also comprise some carbon dioxide, and may also contain trace amounts of carbon monoxide. The remaining components of the product stream may be used as fuel. The quantity of material used as fuel is significantly reduced compared to the original fuel gas. Overall carbon dioxide emissions are therefore reduced compared to combustion of all of the original fuel gas stream (without production of methanol).

The autothermal cracking process from which the fuel gas stream of step (a) of the present invention is derived is generally as described, for example, in EP-332289B; EP-529793B; EP-A-0709446 and WO 00/14035.

In a further embodiment, the present invention provides a process for the production of olefins and methanol comprising
(i) contacting a paraffinic hydrocarbon containing feed and an oxygen-containing gas with a catalyst capable of supporting combustion beyond the normal fuel rich limit of flammability to produce a product stream comprising olefins, hydrogen, carbon monoxide and methane, and
(ii) treating said product stream to separate a fuel gas stream comprising 20-50mol% hydrogen, 20-50mol% carbon monoxide and 20-50mol% methane, which is used for the production of methanol as described herein.

Combustion of a portion of the paraffinic hydrocarbon with oxygen occurs on contact with the autothermal cracking catalyst generating heat. The heat generated drives the subsequent dehydrogenation of further paraffinic hydrocarbon to produce the olefin: Other feed components may be provided, such as hydrogen. Hydrogen combusts to generate heat and thereby reduces the amount of paraffinic hydrocarbon combustion required to generate the required heat for dehydrogenation. The feeds may also be preheated to reduce the amount of heat that need be generated by combustion.

The catalyst capable of supporting combustion beyond the fuel rich limit of flammability usually comprises a Group VIII metal as its catalytic component. Suitable Group VIII metals include platinum, palladium, ruthenium, rhodium, osmium and iridium. Typical Group VIII metal loadings range from 0.01 to 100wt %, preferably, between 0.01 to 20 wt %, and more preferably, from 0.01 to 10 wt % based on the total dry weight of the catalyst.

Where a Group VIII catalyst is employed, it may be employed in combination with one or more catalyst promoters, preferably selected from Group IIIA, IVA, VA and transition metals (the transition metal promoter, if present, being a different metal to that which may be employed as the Group VIII metal catalytic component).

The catalyst may be unsupported, such as in the form of a metal gauze, but is preferably supported. Any suitable support may be used, such as ceramic or metal supports, but ceramic supports are generally preferred. Where ceramic supports are used, the composition of the ceramic support may be any oxide or combination of oxides that is stable at high temperatures of, for example, between 600°C and 1200°C. The support material preferably has a low thermal expansion co-efficient, and is resistant to phase separation at high temperatures.

Suitable ceramic supports include corderite, lithium aluminium silicate (LAS), alumina (α-Al₂O₃), yttria stabilised zirconia, alumina titanate, niascon, and calcium zirconyl phosphate. The ceramic supports may be wash-coated, for example, with γ-Al₂O₃,

The catalyst capable of supporting combustion beyond the fuel rich limit of flammability may be prepared by any method known in the art. For example, gel methods and wet-impregnation techniques may be employed. Typically, the support is impregnated with one or more solutions comprising the metals, dried and then calcined in air. The support may be impregnated in one or more steps. Preferably, multiple impregnation steps are employed. The support is preferably dried and calcined between each impregnation, and then subjected to a final calcination, preferably, in air. The calcined support may then be reduced, for example, by heat treatment in a hydrogen atmosphere.

The oxygen is suitably provided as an oxygen-containing gas. Any suitable molecular oxygen containing gas, such as molecular oxygen itself or air may be used.

Preferred hydrocarbons for autothermal cracking are paraffinic hydrocarbons having at least 2 carbon atoms. For example, the hydrocarbon may be a gaseous hydrocarbon, such as ethane, propane or butane or a liquid hydrocarbon, such as a naphtha or an FT liquid.

Preferably, hydrogen is co-fed. As described above, hydrogen combusts preferentially relative to hydrocarbon, increasing the olefin selectivity of the overall process. The amount of hydrogen combusted may be used to control the amount of heat generated and hence the severity of cracking. Thus, the molar ratio of hydrogen to oxygen can vary over any operable range provided that the ATC product stream comprising olefins is produced. Suitably, the molar ratio of hydrogen to oxygen is in the range 0.2 to 4, preferably, in the range 0.2 to 3.

The preferred stoichiometric ratio of hydrocarbon to oxygen is 5 to 16, preferably, 5 to 13.5 times, preferably, 6 to 10 times the stoichiometric ratio of hydrocarbon to oxygen required for complete combustion of the hydrocarbon to carbon dioxide and water.

Typically the reactants are passed over the catalyst at a pressure dependent gas hourly space velocity of greater than 10,000 h⁻¹ barg⁻¹ preferably greater than 20,000 h⁻¹ barg⁻¹ and, most preferably, greater than 100,000 h⁻¹ barg ⁻¹. For example, at 20 barg pressure, the gas hourly space velocity is most preferably, greater than 2,000,000 h⁻¹. It will be understood, however, that the optimum gas hourly space velocity will depend upon the nature of the feed composition.

The autothermal cracking step may suitably be carried out at a catalyst exit temperature in the range 600°C to 1200°C. Suitably the catalyst exit temperature is at least 720°C such as at least 750°C. Preferably, the autothermal cracking step is carried out at a catalyst exit temperature in the range 850°C to 1050°C and, most preferably, in the range 850°C to 1000°C.

The autothermal cracking step is usually operated at a pressure of greater than 0.5barg, preferably at a pressure of least 10 barg, and more preferably at a pressure of at least 20 barg. The pressure is preferably less than 50 barg, and more preferably less than 35 barg, for example in the range 20 to 30 barg.

The ATC product stream is quenched after it emerges from the reaction (catalyst) zone to avoid further reactions taking place. Usually the product stream is cooled to between 750-600°C within less than 100milliseconds of formation, preferably within 50milliseconds of formation and most preferably within 20milliseconds of formation e.g. within 10milliseconds of formation. The heat from the quenching may be used to generate high-pressure steam, which can be used to provide power for those parts of the overall process requiring it.

The autothermal cracking reaction produces a product stream comprising olefins, hydrogen, carbon monoxide, methane, and small amounts of acetylenes, aromatics and carbon dioxide.

Separation of the desired products from the cooled product stream may be achieved by any suitable technique or series of techniques. Typically water and carbon dioxide are removed first, for example, using an amine wash. The fuel gas stream comprising hydrogen, carbon monoxide and methane according to the present invention may be removed next, typically using a demethaniser. Where not already at high pressure, the product stream may be compressed to a pressure between 15 and 40 barg to facilitate the separation of the fuel gas stream.

Any suitable subsequent treatments to separate olefinic products from the remaining components of the product stream may be used. Typically hydrogenation is used to remove acetylenic compounds and dienes, ethylene will be separated using a deethaniser, and any propylene using a depropaniser.

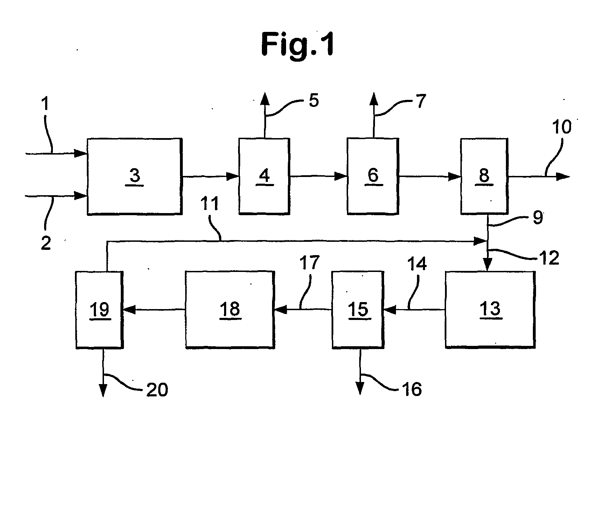

The invention will now be illustrated with respect to Figure 1, which represents, schematically, a preferred process according to the present invention. It should be noted that heating, compression and similar conventional process steps as would be known to the person skilled in the art have been omitted to simplify representation of the key process steps of the invention.

Referring to Figure 1, ethane, 1, and oxygen, 2 are fed to an ATC reactor, 3. The product stream is passed to a quench, 4, from which is separated water and higher hydrocarbons, 5, and then to an amine wash, 6, to remove carbon dioxide and further water, 7. The remaining product stream is then passed to a demethaniser, 8, to remove a fuel gas stream comprising 20-50mol% hydrogen, 20-50mol% carbon monoxide and 20-50mol% methane, 9. The remaining ATC product stream, 10, may be further purified to separate ethylene (not shown), for example, using a deethaniser, to separate C3+ hydrocarbons which can be recycled to the ATC reactor, 3, and to separate ethylene from ethane.

The fuel gas stream comprising 20-50mol% hydrogen, 20-50mol% carbon monoxide and 20-50mol% methane, 9, is combined with a recycle stream, 11 comprising hydrogen, and the combined stream, 12, is compressed and passed to a methanol synthesis reactor, 13, wherein it is contacted with a catalyst for the conversion of synthesis gas to methanol at a pressure in the range 50 to 125 barg, to produce a product stream comprising methanol, carbon monoxide, hydrogen and methane, 14.

The product stream comprising methanol, carbon monoxide, hydrogen, and methane, 14, is subsequently passed to a condenser, 15, to separate a product stream comprising methanol, 16, and a process stream comprising carbon monoxide, hydrogen and methane, 17, which is passed, with additional water, to a water-gas shift reactor, 18, and subsequently to a membrane separator, 19, from which is separated a recycle stream comprising hydrogen, which is recycled to the methanol synthesis reactor as the recycle stream, 11, and a residual stream, 20.

The product stream comprising methanol, 16, is preferably treated to remove traces of hydrogen, carbon monoxide, carbon dioxide and methane to provide a purified methanol stream (not shown).

The residual stream, 20, typically comprises hydrogen, carbon monoxide, carbon dioxide, methane and water. It is preferably treated to remove water, and the remainder of the stream may be used as a fuel gas (not shown).

### Example

The methanol synthesis and recycle processes were modelled using an ASPEN flowsheet, the principle steps of which are as shown in Figure 1. The compositions of the principle streams are given in Table 1.

**Table 1**

| Stream | 9 | 11 | 12 | 14 | 16 | 17 | 20 |
|---|---|---|---|---|---|---|---|
| Mass flow rate (kg/hr) | 27600 | 3581 | 31181 | 31181 | 11628 | 19553 | 28583 |
| Composition (mol%) | | | | | | | |
| Hydrogen | 36 | 90 | 48 | 25 | 0.7 | 34 | 8.5 |
| CO | 29 | 0 | 22 | 13 | 0.4 | 14 | 0.5 |
| CO₂ | 0 | 10 | 3 | 1 | 1.1 | 4 | 11.5 |
| Methane | 35 | 0 | 27 | 38 | 2.7 | 48 | 44 |
| Methanol | 0 | 0 | 0 | 21 | 94.7 | 1 | 1 |
| H₂O | 0 | 0 | 0 | 2 | 0.4 | 0 | 34.5 |

As well as producing methanol, it can be seen that the quantity of hydrogen and carbon monoxide for use as fuel is significantly reduced compared to the original fuel gas.

## Claims

1. A process for the production of methanol, said process comprising:
(a) providing a fuel gas stream comprising 20-50mol% hydrogen, 20-50mol% carbon monoxide and 20-50mol% methane, which fuel gas stream has been separated from the product stream obtained from a process for the autothermal cracking of paraffinic hydrocarbons with oxygen in the in the presence of a catalyst capable of supporting combustion beyond the fuel rich limit of flammability,
(b) contacting the fuel gas stream comprising 20-50mol% hydrogen, 20-50mol% carbon monoxide and 20-50mol% methane obtained from step (a) with a catalyst for the conversion of synthesis gas to methanol at a pressure in the range 50 to 125 barg, to produce a product stream comprising methanol, carbon monoxide, hydrogen and methane,
(c) treating the product stream comprising methanol, carbon monoxide, hydrogen, and methane from step (b) to separate a product stream comprising methanol and a process stream comprising carbon monoxide, hydrogen and methane,
(d) contacting the process stream comprising carbon monoxide, hydrogen and methane from step (c) with a water-gas shift catalyst to convert at least some of the carbon monoxide to produce additional hydrogen, and
(e) separating at least a portion of the hydrogen and recycling to step (b).

2. A process as claimed in claim,1, wherein the fuel gas stream in step (a) comprises 30-40mol%, hydrogen, 25-35mol% carbon monoxide and 30-40mol% methane.

3. A process as claimed in claim 1 or claim 2, wherein the fuel gas stream in step (a) comprises a molar ratio of hydrogen to carbon monoxide in the range 1:1 to 1.5:1.

4. A process as claimed in any one of the preceding claims wherein the fuel gas stream is used directly as separated from the autothermal cracking product stream without any additional treatment steps.

5. A process as claimed in any one of the preceding claims wherein the hydrogen recycled from step (e) acts to increase the hydrogen to carbon monoxide ratio of the fuel gas such that the reactants with which the catalyst is actually contacted in step (b) have approximately the stoichiometric 2:1 molar ratio of hydrogen to carbon monoxide for methanol production.

6. A process as claimed in any one of the preceding claims wherein carbon dioxide is recycled with the hydrogen from step (e).

7. A process as claimed in any one of the preceding claims wherein in step (e) the hydrogen is separated by membrane separation or pressure swing absorption.

## Patentansprüche

1. Verfahren zur Herstellung von Methanol, wobei das Verfahren:
(a) das Bereitstellen eines Brenngasstroms, umfassend 20 - 50 mol-% Wasserstoff, 20 - 50 mol-% Kohlenmonoxid und 20 - 50 mol-% Methan, wobei der Brenngasstrom aus dem Produktstrom, erhalten aus einem Verfahren zum autothermen Kracken von paraffinischen Kohlenwasserstoffen mit Sauerstoff in Gegenwart eines Katalysators, der die Verbrennung über die brennstoffreiche Entflammbarkeitsgrenze hinaus unterhalten kann, abgetrennt worden ist;
(b) das Kontaktieren des in Schritt (a) erhaltenen Brenngasstroms, umfassend 20-50 mol-% Wasserstoff, 20 - 50 mol-% Kohlenmonoxid und 20 - 50 mol-% Methan, mit einem Katalysator für die Umwandlung des Synthesegases in Methanol bei einem Druck im Bereich von 50 bis 125 barg, um so einen Produktstrom zu erzeugen, der Methanol, Kohlenmonoxid, Wasserstoff und Methan umfaßt,
(c) das Behandeln des Produktstromes aus Schritt (b), der Methanol, Kohlenmonoxid, Wasserstoff und Methan umfaßt, um einen Produktstrom, der Methanol umfaßt, und einen Verfahrensstrom, der Kohlenmonoxid, Wasserstoff und Methan umfaßt, abzutrennen;
(d) das Kontaktieren des Verfahrensstroms aus Schritt (c), der Kohlenmonoxid, Wasserstoff und Methan umfaßt, mit einem Konvertierungskatalysator, um so zur Erzeugung von weiterem Wasserstoff zumindest etwas von dem Kohlenmonoxid umzuwandeln, und
(e) das Abtrennen zumindest eines Teils des Wasserstoffes und das Rückführen in Schritt (b)
umfaßt.

2. Verfahren nach Anspruch 1, wobei der Brenngasstrom in Schritt (a) 30 - 40 mol-% Wasserstoff, 25 - 35 mol-% Kohlenmonoxid und 30 - 40 mol-% Methan umfaßt.

3. Verfahren nach Anspruch 1 oder Anspruch 2, wobei der Brenngasstrom in Schritt (a) ein Molverhältnis von Wasserstoff zu Kohlenmonoxid im Bereich von 1 : 1 bis 1,5 : 1 umfaßt.

4. Verfahren nach einem der vorhergehenden Ansprüche, wobei der Brenngasstrom direkt wie aus dem autothermen Krackproduktstrom getrennt ohne weitere Behandlungsschritte verwendet wird.

5. Verfahren nach einem der vorhergehenden Ansprüche, wobei der aus Schritt (e) rückgeführte Wasserstoff das Wasserstoff-zu-Kohlenmonoxid-Verhältnis des Brenngases so erhöht, daß die Reaktanten, mit denen der Katalysator in Schritt (b) tatsächlich kontaktiert wird, un gefähr das stöchiometrische 2 : 1 -Molverhältnis von Wasserstoff zu Kohlenmonoxid für die Methanolherstellung haben.

6. Verfahren nach einem der vorhergehenden Ansprüche, wobei Kohlendioxid mit dem Wasserstoff aus Schritt (e) rückgeführt wird.

7. Verfahren nach einem der vorhergehenden Ansprüche, wobei in Schritt (e) der Wasserstoff durch Membrantrennung oder Druckschwingabsorption abgetrennt wird.

## Revendications

1. Procédé de production de méthanol, ledit procédé comprenant les étapes consistant à :
(a) fournir un flux de gaz combustibles comprenant de 20 à 50 % en moles d'hydrogène, de 20 à 50 % en moles de monoxyde de carbone et de 20 à 50 % en moles de méthane, ledit flux de gaz combustibles ayant été séparé du produit se présentant sous la forme d'un flux obtenu à partir d'un processus de craquage autothermique d'hydrocarbures paraffiniques avec de l'oxygène en présence d'un catalyseur capable d'entretenir la combustion au-delà de la limite de flammabilité d'un flux riche en combustible,
(b) mettre en contact le flux de gaz combustibles comprenant de 20 à 50 % en moles d'hydrogène, de 20 à 50 % en moles de monoxyde de carbone et de 20 à 50 % en moles de méthane obtenu à l'étape (a) avec un catalyseur en vue de la conversion du gaz de synthèse en méthanol sous une pression se situant dans une fourchette de 50 à 125 bars, pour produire un produit se présentant sous la forme d'un flux comprenant du méthanol, du monoxyde de carbone, de l'hydrogène et du méthane,
(c) traiter le produit se présentant sous la forme d'un flux comprenant du méthanol, du monoxyde de carbone, de l'hydrogène et du méthane de l'étape (b) de façon à séparer un produit se présentant sous la forme d'un flux comprenant du méthanol et un flux propre au processus comprenant du monoxyde de carbone, de l'hydrogène et du méthane,
(d) mettre en contact le flux propre au processus comprenant du monoxyde de carbone, de l'hydrogène et du méthane de l'étape (c) avec un catalyseur de réaction de gaz à l'eau de façon à convertir au moins une partie du monoxyde de carbone en vue de produire davantage d'hydrogène, et
(e) isoler au moins une partie de l'hydrogène en vue d'un recyclage dans le cadre de l'étape (b).

2. Procédé selon la revendication 1, dans lequel le flux de gaz combustibles de l'étape (a) comprend de 30 à 40 % en moles d'hydrogène, de 25 à 35 % en moles de monoxyde de carbone et de 30 à 40 % en moles de méthane.

3. Procédé comme revendiqué dans la revendication 1 ou la revendication 2, dans lequel le flux de gaz combustibles de l'étape (a) est **caractérisé par** un rapport molaire entre l'hydrogène et le monoxyde de carbone se situant dans un intervalle de 1 à 1 à 1,5 à 1.

4. Procédé comme revendiqué dans l'une quelconque des revendications précédentes, dans lequel le flux de gaz combustibles est directement utilisé à l'issue de sa séparation à partir du produit de craquage autothermique se présentant sous la forme d'un flux sans aucune étape de traitement supplémentaire.

5. Procédé comme revendiqué dans l'une quelconque des revendications précédentes, dans lequel l'hydrogène recyclé de l'étape (e) a pour fonction d'entraîner une augmentation du rapport entre l'hydrogène et le monoxyde de carbone dans le flux de gaz combustibles de façon à ce que les réactants avec lesquels le catalyseur est effectivement mis en contact à l'étape (b) présentent un rapport molaire stoechiométrique d'environ 2 à 1 entre l'hydrogène et le monoxyde de carbone pour la production de méthanol.

6. Procédé comme revendiqué dans l'une quelconque des revendications précédentes, dans lequel le dioxyde de carbone est recyclé avec l'hydrogène de l'étape (e).

7. Procédé comme revendiqué dans l'une quelconque des revendications précédentes, dans lequel, au cours de l'étape (e), l'hydrogène est isolé par séparation sur membrane ou absorption par variation de pression.
